(19) Europäisches Patentamt
European Patent Office
Office européen des brevets

(11) **EP 1 012 204 B1**

(12) **EUROPÄISCHE PATENTSCHRIFT**

(45) Veröffentlichungstag und Bekanntmachung des Hinweises auf die Patenterteilung:
**29.01.2003 Patentblatt 2003/05**

(21) Anmeldenummer: **98948849.9**

(22) Anmeldetag: **20.08.1998**

(51) Int Cl.⁷: **C08J 3/14**, A61K 9/16
// (C08L3/12, 5:00)

(86) Internationale Anmeldenummer:
**PCT/EP98/05297**

(87) Internationale Veröffentlichungsnummer:
**WO 99/011695 (11.03.1999 Gazette 1999/10)**

(54) **SPHÄRISCHE LINEARE POLYSACCHARIDE ENTHALTENDE MIKROPARTIKEL**

SPHERICAL MICROPARTICLES CONTAINING LINEAR POLYSACCHARIDES

MICRO PARTICULES SPHERIQUES CONTENANT DES POLYSACCHARIDES LINEAIRES

(84) Benannte Vertragsstaaten:
**BE DE ES FR GB IT NL**

(30) Priorität: **28.08.1997 DE 19737481**

(43) Veröffentlichungstag der Anmeldung:
**28.06.2000 Patentblatt 2000/26**

(73) Patentinhaber: **Celanese Ventures GmbH**
**65926 Frankfurt am Main (DE)**

(72) Erfinder:
• **BENGS, Holger**
  **D-60598 Frankfurt am Main (DE)**
• **GRANDE, Jürgen**
  **D-65812 Bad Soden (DE)**
• **SCHNELLER, Arnold**
  **D-64409 Messel (DE)**
• **BÖHM, Gitte**
  **D-60439 Frankfurt am Main (DE)**

(74) Vertreter: **Luderschmidt, Schüler & Partner**
**Patentanwälte**
**Industriepark Höchst**
**65926 Frankfurt (DE)**

(56) Entgegenhaltungen:
**EP-B- 0 476 063          EP-B- 0 648 115**
**WO-A-88/08011            DE-A- 2 737 947**
**DE-A- 4 120 760          GB-A- 2 247 242**
**US-A- 5 576 015**

• **DATABASE WPI Week 8025 Derwent Publications Ltd., London, GB; AN 10509C XP002089601 & JP 55 001244 B (KAKEN YAKU KAKO) , 12. Januar 1980 & CHEMICAL ABSTRACTS, vol. 94, no. 12, 23. März 1981 Columbus, Ohio, US; abstract no. 90331, HAYASHI ICHIRO ET AL.: "Inclusion compounds of amylose with drugs"**
• **DATABASE WPI Week 7625 Derwent Publications Ltd., London, GB; AN 13758X XP002089602 & JP 51 001372 A (SUMITOMO CHEM CO LTD) , 8. Oktober 1976 & CHEMICAL ABSTRACTS, vol. 84, no. 20, 17. Mai 1976 Columbus, Ohio, US; abstract no. 136876, MIYAMOTO MASATOSHI ET AL.: "Microencapsulation of pullulan or hydroxypropyl pullulan."**

**Beschreibung**

Die Erfindung betrifft sphärische Mikropartikel, welche lineare Polysaccharide enthalten, Verfahren zu deren Herstellung sowie deren Verwendung, insbesondere bei der kontrollierten Abgabe von Wirkstoffen.

Verfahren zur Herstellung von Partikeln, insbesondere Mikropartikeln aus Polymeren, wie z.B. Polysacchariden, für verschiedenste Anwendungen sind recht komplizierte Verfahren, die eine genaue Einhaltung unterschiedlicher Parameter voraussetzen. Insbesondere führen viele Verfahren auch nur zu geringen Ausbeuten und zu sehr breiten Partikelverteilungen. Zu nennen in diesem Zusammenhang sind vor allem Sprühtrocknung, Phasengrenzflächenkondensation und Emulsionsverfahren (z.B. WO-Verfahren = Wasser in Öl Emulsionen, WOW = Wasser in Öl in Wasser Emulsionen, Koazervation, Phasenseparation, Dispersion). Insbesondere Emulsionsverfahren, aber auch Sprühtrocknungen aus Zweiphasensystemen, erfordern ein sehr exaktes Vorgehen und in der überwiegenden Zahl der Fälle die Verwendung von Hilfsmitteln (Emulgatoren). Stabile Emulsionen sind oftmals nur mit hohem Aufwand und einer präzisen Kontrolle einer Vielzahl von Parametern (Temperatur, Rührgeschwindigkeit usw.) herzustellen, und die umfassende Abtrennung der Partikel bereitet Probleme. Die Ausbeute an Partikeln ist oft sehr niedrig, insbesondere ist die Einschlußrate von Wirksubstanzen ungenügend. Ein Aspekt, der im Fall teurer Pharmawirkstoffe die Anwendung einer Technologie verhindern kann.

Kugelförmige Mikropartikel, die neben Weinsäure enthaltenden Polykondensaten auch Ethylstärke oder sonstige Polysaccharide enthalten können, werden gemäß US-PS 5 391 696 einmal nach dem Verfahren der Sprühtrocknung erhalten, mit dem jedoch die Teilchengröße und besonders die Größenverteilung nur sehr schwer zu regeln ist. Eine weitere in dieser Patentschrift beschriebene Möglichkeit ist das Auflösen des Polymers in einem Lösungsmittel oder Lösungsmittelgemisch und das Eintropfenlassen der Lösung in ein kaltes verflüssigtes Gas, z.B. flüssigen Stickstoff, wobei sich kugelförmige Partikel bilden. Die Kügelchen können dann in Wasser eingebracht werden, das gleichzeitig das Polymer ausfällt und das Lösungsmittel extrahiert. Dieses Verfahren ist umständlich, aufwendig und unökonomisch. Auch läßt die Gleichmäßigkeit der Partikeldimensionen zu wünschen übrig.

Die EP-B1-0 251 476 beschreibt die Herstellung von Mikropartikeln aus Polylactiden, in denen ein makromolekulares Polypeptid dispergiert ist. Auch hier ist eine intensive Kontrolle der verschiedensten Parameter erforderlich. Einheitliche sphärische Teilchen werden nicht erhalten.

Mikropartikel, welche Wirkstoffe und Gase enthalten, werden in der WO 95/07 072 beschrieben. Die Herstellung erfolgt nach aufwendigen Emulsionsverfahren, die Größenverteilung der Partikel ist sehr uneinheitlich.

Yu Jiugao und Liu Jie berichten in starch/stärke 46(7)252-5(1994) über die Effekte der Suspensions-Vernetzungs-Reaktionsbedingungen auf die Größe von Stärke-Mikrokugeln. Die Vernetzung findet in drei Stufen statt; das Medium ist eine Wasserin-Öl Suspension, als Öl-Phase dient ein Erdnußöl/Toluol Gemisch. Vorgelatinisierte Stärke wird als wässrige Lösung, die noch Natriumhydroxid und Ethylendiamintetraessigsäure enthält, zugegeben. Ferner ist die Gegenwart eines oberflächenaktiven Mittels bzw. Stabilisators erforderlich.

Von Nachteil bei dem dort beschriebenen Verfahren ist, daß das Ergebnis von einer Vielzahl von Faktoren abhängig ist, nämlich von der Dichte, der Viskosität und den Konzentrationsverhältnissen sowohl der wäßrigen als auch der Öl-Phase, vom Stabilisator und von der Rührgeschwindigkeit, außerdem ist die Anwesenheit des Stabilisators nachteilig. Zudem ist es schwierig die Vielzahl der gegebenen Parameter zu kontrollieren, so daß die Reproduzierbarkeit nicht zufriedenstellend ist.

Mit makromolekularen Wirkstoffen beladene Teilchen aus wasserunlöslichen Polymeren wie Polymilchsäure oder Ethylcellulose werden entsprechend der Lehre der EP-B1-0 204 476 erhalten, indem man den partikulären Wirkstoff in einer acetonischen Lösung des Polymeren suspendiert und das Lösungsmittel bei Raumtemperatur abdampft. Die dabei entstehenden Teilchen zeigen noch nicht die gewünschten pharmakologischen Effekte, so daß eine Weiterverarbeitung zu sogenannten Pellets notwendig ist.

Obwohl bereits Mikropartikel mit sphärischer Gestalt sowie Verfahren zu deren Herstellung bekannt sind, besteht noch ein Bedürfnis nach derartigen Mikropartikeln mit verbesserten Eigenschaften sowie nach vorteilhafteren, insbesondere ökonomischen und leicht reproduzierbaren, Herstellungsverfahren. Aufgabe der Erfindung ist es deshalb, Mikropartikel zur Verfügung zu stellen, die eine weitgehend regelmäßige sphärische Gestalt besitzen, die ferner eine möglichst enge Größenverteilung, d.h. eine große Einheitlichkeit aufweisen und die sich vielseitig verwenden lassen. Aufgabe der Erfindung ist weiter, ein Verfahren zur Herstellung derartiger Mikropartikel zur Verfügung zu stellen, das einfach und wirtschaftlich durchzuführen ist, das Mikropartikel mit regelmäßigen Strukturen und großer Einheitlichkeit liefert, die gute mechanische Eigenschaften besitzen, die biologisch abbaubar sind, die mit verschiedensten Wirkstoffen versehen werden können und die besonders geeignet sind für die kontrollierte Abgabe von Wirkstoffen.

Diese Aufgabe wird gelöst durch sphärische Mikropartikel mit einem mittleren Durchmesser von 1 nm bis 100 μm, bestehend ganz oder teilweise aus mindestens einem wasserunlöslichen, linearen Polysaccharid, wobei die Partikel eine Dispersität in einem Bereich von 1,0 bis 10,0 aufweisen und separiert vorliegen.

Unter sphärischen Mikropartikeln sind Mikropartikel, die annähernd Kugelform besitzen, zu verstehen. Bei Beschreibung einer Kugel durch von einem gemeinsamen Ursprung ausgehende, in den Raum gerichtete Achsen gleicher Länge, die den Radius der Kugel in allen Raumrichtungen definieren, ist für die sphärischen Mikropartikel eine Abweichung der Achsenlängen vom Idealzustand der Kugel von 1% bis 40% möglich. Bevorzugt werden sphärische Mikropartikel mit Abweichungen bis 25%, besonders bevorzugt bis 15% erhalten. Die Oberfläche der sphärischen Mikropartikel kann makroskopisch mit der einer Himbeere verglichen werden, wobei die Tiefe der "Eindellungen" oder "Einschnitte" maximal 20% des mittleren Durchmessers der sphärischen Mikropartikel betragen soll.

"Lineare, wasserunlösliche Polysaccharide" im Sinne der vorliegenden Erfindung sind Polysaccharide, die aus Monosacchariden, Disacchariden oder anderen monomeren Bausteinen derart aufgebaut sind, daß die Monosaccharide, Disaccharide oder anderen monomeren Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind die beiden Grundeinheiten, die den Anfang und das Ende des Polysaccharids bilden. Diese Grundeinheiten haben nur eine Verknüpfung zu einem weiteren Monomer. Bei drei Verknüpfungen (kovalente Bindungen) spricht man von einer Verzweigung. Lineare, wasserunlösliche Polysaccharide im Sinne der Erfindung weisen keine Verzweigungen oder allenfalls nur in untergeordnetem Maß auf, so daß sie bei sehr kleinen Verzweigungsanteilen den herkömmlichen analytischen Methoden nicht zugänglich sind.

Unter dem Begriff "wasserunlösliche Polysaccharide" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag GmbH, Frankfurt, 9. Auflage, 1987) entsprechend den Klassen 4 bis 7 unter die Kategorien "wenig löslich", "schwer lösliche", "sehr schwer lösliche" bzw. "praktisch unlösliche" Verbindungen fallen.

Im Rahmen der Erfindung werden bevorzugt lineare, wasserunlösliche Polysaccharide, welche in einem biotechnischen, insbesondere in einem biokatalytischem, auch biotransformatorischem, oder einem fermentativen Prozeß hergestellt wurden.

Lineare Polysaccharide hergestellt durch Biokatalyse (auch: Biotransformation) im Rahmen dieser Erfindung bedeutet, daß das lineare Polysaccharid durch katalytische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird.

Linerare Polysaccharide aus Fermentationen sind im Sprachgebrauch der Erfindung lineare Polysaccharide, die durch fermentative Prozesse unter der Verwendung in der Natur vorkommende Organismen, wie Pilzen, Algen oder Bakterien oder unter der Verwendung von in der Natur nicht vorkommender Organismen, aber unter Zuhilfenahme von gentechnischen Methoden allgemeiner Definition modifizierten natürlichen Organismen, wie Pilzen, Algen oder Bakterien gewonnen werden oder unter Einschaltung und Mithilfe von fermentativen -Prozessen gewonnen werden können.

Lineare Polymere gemäß der vorliegenden Erfindung können neben dem bevorzugten 1,4-$\alpha$-D-Polyglucan auch weitere Polyglucane oder andere lineare Polysaccharide wie etwa Pektine, Mannane oder Polyfructane sein.

Darüber hinaus können lineare Polymere zur Herstellung der in der vorliegenden Erfindung beschriebenen Mikropartikel auch aus der Reaktion weiterer nicht-linearer Polysaccharide dadurch gewonnen werden, daß nichtlineare Polysaccharide, die Verzweigungen enthalten, derart mit einem Enzym behandelt werden, daß es zur Spaltung der Verzweigungen kommt, so daß nach ihrer Abtrennung lineare Polysaccharide vorliegen. Bei diesen Enzymen kann es sich beispielsweise um Amylasen, iso-Amylasen, Gluconohydrolasen oder Pullulanasen handeln.

In einer besonders vorteilhaften Ausführungsform der Erfindung bestehen die sphärischen Mikropartikel ganz oder teilweise aus 1,4-$\alpha$-D-Polyglucan. Bevorzugt wurde das 1,4-$\alpha$-D-Polyglucan mittels eines biokatalytischen (biotransformatorischen) Prozesses mit Hilfe von Polysaccharidsynthasen oder Stärkesynthasen oder Glykosyltransferasen oder $\alpha$-1,4-Glucantransferasen oder Glycogensynthasen oder Amylosucrasen oder Phosphorylasen hergestellt. Die Molekulargewichte $M_w$ der erfindungsgemäß verwendeten linearen Polysaccharide können in einem weiten Bereich von $10^3$ g/mol bis $10^7$ g/mol variieren. Für das vorzugsweise verwendete lineare Polysaccharid 1,4-$\alpha$-D-Polyglucan werden vorzugsweise Molekulargewichte $M_w$ im Bereich von $10^4$ g/mol bis $10^5$ g/mol, insbesondere 2 x $10^4$ g/mol bis 5 x $10^4$ g/mol verwendet.

Es wurde nun überraschend festgestellt, daß durch ein sehr einfaches Verfahren sehr einheitliche Mikropartikel aus wasserunlöslichen linearen Polysacchariden in großen Mengen hergestellt werden können, die so mit ähnlichen kommerziell erhältlichen Polysacchariden, wie etwa Amylose oder Stärke, nicht erhalten werden.

Gegenstand der Erfindung ist daher ferner ein Verfahren zur Herstellung von sphärischen Mikropartikeln, welche ganz oder teilweise aus wasserunlöslichen, linearen Polysacchariden, insbesondere 1,4-$\alpha$-D-Polyglucan bestehen durch Lösen des wasserunlöslichen, linearen Polysaccharids oder des 1,4-$\alpha$-D-Polyglucan in einem Lösungsmittel, Einbringen der Lösung in ein Fällmittel, Kühlen des dabei entstehenden Gemisches und Abtrennen der gebildeten Mikropartikel. In den Ansprüchen 20 bis 23 werden besonders vorteilhafte Ausführungsformen des erfindungsgemäßen Verfahren angegeben.

In einer weiteren vorteilhaften Ausführungsform wurden die linearen, wasserunlöslichen Polysaccharide durch enzymatische Behandlung von verzweigten oder hochverzweigten Polysacchariden hergestellt.

Dimethylsulfoxid ist bevorzugtes Lösungsmittel für die Auflösung der linearen Polysaccharide; weitere Lösungsmittel können u.a. sein: Formamid, Acetamid, N,N-Dimethyl-formamid, N,N-Dimethylacetamid, N-Methylmorpholin-N-oxid in Gegenwart von Wasser sowie weitere N-substituierte Morpholin-N-oxide, wäßrige Lösung mit hohem oder niedrigem pH-Wert.

Als Fällungsmittel ist Wasser bevorzugt; der Prozeß kann durch die Verwendung anderer Lösungsmittel die Wasser ganz oder teilweise ersetzen können, z.B. Dichlormethan beeinflußt werden, wobei u.a. die Dauer des Fällprozesses und die Struktur der Oberfläche der Partikel gesteuert werden können.
Auch Gemische von Wasser mit Alkoholen, z.B. Methanol, Ethanol, Isopropanol, sind dazu geeignet, die Prozeßparameter, sowie die Eigenschaften der Partikel zu beeinflussen.

Die Temperatur während des Fällprozesses liegt im allgemeinen vorzugsweise im Bereich von 0°C bis - 10°C, es können jedoch auch höhere oder tiefere Temperaturen genommen werden.

Der Fällprozeß kann relativ langsam bei tiefer Temperatur über Nacht durchgeführt werden oder durch Variation des Fällungsmittels und der Temperatur beeinflußt werden.

Durch Mitverwendung geeigneter Zusatzstoffe läßt sich Einfluß nehmen auf die Eigenschaften der Partikel wie die Größe, die Struktur der Oberfläche usw. sowie auf die Prozeßführung. Geeignete Zusatzstoffe sind beispielsweise oberflächenaktive Stoffe wie Natriumdodecylsulfat, oder N-Methylgluconamid, Zucker, z.B. Fructose, Saccharose, Glucose.

Die oberflächenaktiven Substanzen können anionischer, kationischer oder nichtionischer Natur sein.

Generelle Beispiele oberflächenaktiver Substanzen sind:

Polysorbate (z.B. Tween®), Alkylpolyglycolether, Ethylenoxid-Propylenoxid-Blockpolymere (z.B. Pluronic®), Alkylpolyglycolethersulfate, Alkylsulfate (z.B. das bereits erwähnte Natriumdodecylsulfat), Fettsäureglycolester. Die Zusatzstoffe werden bevorzugt dem Fällungsmittel zugefügt.

Die Konzentration des linearen Polysaccharids in der Lösung kann in weiten Grenzen variiert werden, sie beträgt vorzugsweise 0,1 g Polysaccharid pro 1 ml Lösungsmittel.

Andere Bereiche wie 0,05 g/ml bis 0,2 g/ml oder 0,02 g/ml bis 0,5 g/ml sind möglich.

Die erfindungsgemäßen Partikel können aus mindestens einem linearen Polysaccharid bestehen und können mindestens eine Wirksubstanz enthalten. Die Oberfläche kann glatt oder rauh sein.
Die Mikropartikel können aus einer einzigen linearen Polysaccharidsubstanz, insbesondere 1,4-$\alpha$-D-Polyglucan aufgebaut sein. Es ist aber auch möglich, ein anderes lineares wasserunlösliches Polysaccharid beizumischen. Auch können andere Polymere, insbesondere andere biokompatible oder biologisch abbaubare Polymere mitverwendet werden. Dabei hängt die Menge des oder der anderen Polymeren, die beigemengt werden kann, ohne daß die sphärische Gestalt und sonstige gute Eigenschaften der Mikropartikel nachteilig verändert werden, stets von dem zugesetzten Polymer ab. Sie kann bei bis zu 10% und darüber liegen, in bestimmten Fällen auch darunter. Die noch vertretbare maximale Menge kann leicht durch wenige Mischversuche bestimmt werden.

Die Partikel können mittlere Durchmesser (Zahlenmittelwert) aufweisen wie 1 nm bis 100 µm, bevorzugt 100 nm bis 10 µm, besonders bevorzugt 1 µm bis 3 µm.

Die Partikel zeigen eine Charakteristik der Durchmesser $d_w$ zu $d_n$ von (Dispersität)
1,0 bis 10,0,
bevorzugt 1,5 bis 5,0,
besonders bevorzugt 2,0 bis 2,6

$d_n$ = Zahtenmittelwert des Durchmessers
$d_w$ = Gewichtsmittelwert des Durchmessers

Die hier benutzten Mittelwerte definieren sich wie folgt:

$d_n = \Sigma\, n_i \times d_i\, /\, \Sigma\, n_i$ = Zahlenmittelwert
$d_w = \Sigma\, n_i \times d_i^2 /\, \Sigma\, n_i \times d_i$ = Gewichtsmittelwert

$n_i$ = Anzahl der Partikel mit dem Durchmesser $d_i$,
$d_i$ = ein bestimmter Durchmesser,
i = fortlaufender Parameter.

Der Begriff "Gewicht" steht hier nicht für Masse, sondern für ein gewichtetes Mittel. Die größeren Durchmesser erhalten einen höheren Stellenwert; durch den Exponenten 2 werden Durchmesser größerer Partikel stärker gewichtet.

Die Dispersität der Verteilung der Durchmesser bei den Partikeln ist definiert als:

$$D = d_w/d_n$$

Die Uneinheitlichkeit der Verteilung der Durchmesser ist definiert als:

$$U = d_w/d_n - 1 = D - 1$$

Je näher der Wert für die Uneinheitlichkeit bei "0" liegt, desto einheitlicher sind die Partikel hinsichtlich der Verteilung ihrer Größe geformt.

Die Mikropartikel können besonders auch wegen ihrer einheitlichen Gestalt und Größe in verschiedenen Anwendungsbereichen, entweder als solche in reiner Form oder dadurch, daß Wirksubstanzen im weitesten Sinn eingeschlossen sind, vorteilhaft eingesetzt werden, so z.B.

- als Additive für die Kosmetik in Salben, Pudern, Cremes, Pasten etc.,
- als Träger für Wirksubstanzen in pharmazeutischen und anderen Anwendungen,
- als Glättungsmittel, z.B. zum Verschließen von Poren oder Glätten von Graten,
- als Lebensmittelzusatzstoff, z.B. als Füllkomponente oder zum Verbessern von rheologischen Eigenschaften,
- als Additiv zur Veredelung von z.B. Emulsionspolymerisaten,
- als Trennhilfen, z.B. in der Abtrennung von Verunreinigungen,
- als Verkapselungsmaterial,
- als Träger für magnetische Partikel,
- als Füllmittel für bioabbaubare Polymere oder technische Polymere zur Eigenschaftskontrolle,
- als Additiv zur Eigenschaftskontrolle, z.B. der Porosität, des Gewichts, der Farbe usw.,
- als Partikelstandard zur Eichung oder Bestimmung der Partikelgröße unbekannter Materialien.

Einzelne Wirksubstanzen oder Wirkstoffkombinationen können z.B. folgender Aufstellung entnommen werden:

Pharmazeutische Wirkstoffe, Medikamente, Arzneistoffe, Peptide, Proteine, Nucleinsäuren, Vakzine, Antikörper, Steroide, Oligonucleotide, Aromen, Duftstoffe, Dünger, agritechnische Wirksubstanzen wie Pestizide, Herbizide, Insektizide, Fungizide, Chemikalien mit speziellen Eigenschaften wie Leuchtstoffe, Emulgatoren, Tenside, Pigmente, Oxidationsmittel, Reduktionsmittel, Fullerene, magnetische Komplexe, z.B. paramagnetische Verbindungen.

Somit ist ein weiterer Gegenstand der Erfindung die Verwendung der vorstehend beschriebenen Mikropartikel zur kontrollierten z.B. einer retardierten Abgabe von Wirkstoffen.

Bei dem Verfahren handelt es sich um eine sehr einfache Vorgehensweise. Die Parameter zur Herstellung der Partikel können in weiten Bereichen vorgegeben werden wie Verhältnis Lösungsmittel zu Fällungsmittel, Temperatur während des Ausfällprozesses, Konzentration der Lösung, Geschwindigkeit der Zugabe der Lösung zum Fällungsmittel.

Die Partikel zeichnen sich durch eine hohe Einheitlichkeit hinsichtlich ihrer Größe und der Verteilung ihrer Durchmesser aus.

Durch die Wasserunlöslichkeit des Ausgangspolymeren z.B. 1,4-$\alpha$-D-Polyglucan lassen sich besonders vorteilhaft Anwendungen verwirklichen, die nicht auf eine schnelle Zerstörung der Mikropartikel aus sind und daher auch besonders vorteilhaft in Produkten verwendet werden können, in denen Wasser als eine weitere Komponente enthalten ist.

Die Mikropartikel zeichnen sich durch die Fähigkeit aus, daß sie einer hohen mechanischen Belastbarkeit ausgesetzt werden können.

Insbesondere wirken die Partikel aufgrund ihrer Morphologie und Einheitlichkeit glättend, z.B. von Poren.

Das bevorzugt zum Einsatz gelangende 1,4-$\alpha$-D-Polyglucan kann auf verschiedene Weisen hergestellt werden. Eine sehr vorteilhafte Methode wird in der WO 95/31 553 beschrieben. Auf die Offenbarung in dieser Schrift wird sich hier ausdrücklich bezogen.

Die Erfindung wird anhand der folgenden Beispiele näher erläutert.

Beispiel 1

Herstellung von Mikropartikeln aus 1,4-α-D-Polyglucan.

500 mg 1,4-α-D-Polyglucan werden in 2,5 ml Dimethylsulfoxid (DMSO, p.a. von Riedel-de-Haen) bei ca. 70°C gelöst. Die DMSO-Lösung wird in 100 ml bidestilliertem Wasser unter Rühren eingetropft und die Lösung über Nacht bei 5°C aufbewahrt. Die feine milchige Suspension wird für 15 Minuten bei 3500 Umdrehungen pro Minute zentrifugiert und der Überstand abdekantiert. Der Bodensatz wird mit bidestilliertem Wasser aufgeschlämmt und erneut zentrifugiert. Der Vorgang wird noch zwei Mal wiederholt. Die Suspension wird im Anschluß gefriergetrocknet. Es werden 311 mg weißer 1,4-α-D-Polyglucan Partikel erhalten. Dies entspricht einer Ausbeute von 62% farbloser Mikropartikel.

Beispiel 2

Versuch zur Herstellung von Mikropartikeln aus Amylose, die aus Pflanzen separiert wurde.

500 mg Amylose (aus Kartoffeln von EGA-Chemie) werden in 2,5 ml Dimethylsulfoxid (DMSO, p.a. von Riedel-de-Haen) bei ca. 70°C gelöst. Die DMSO-Lösung ist hochviskos. Sie wird unter Rühren in 100 ml bidestilliertem Wasser gegeben und die Lösung wird über Nacht bei 5°C aufbewahrt. Es bildet sich eine weiße flockige Suspension aus. Die weitere Aufarbeitung erfolgt wie in Beispiel 1 beschrieben. Es werden 210,3 mg eines weißen Feststoffs erhalten (42% Ausbeute), bei dem es sich um nicht-partikuläre Strukturen handelt.

Beispiel 3

Versuch zur Herstellung von Mikropartikeln aus Amylose, die aus Pflanzen separiert wurde.

Dieser Versuch wird analog zu Beispiel 2 durchgeführt. Es werden 500 mg Amylose der Firma Merck (Herstellerangabe: "Amylose für biochemische Zwecke") eingesetzt. Nach der Standzeit über Nacht hat sich eine weiße flockige Suspension ausgebildet. Die weitere Aufarbeitung erfolgt wie in Beispiel 1 beschrieben. Es werden 60 mg eines weißen Feststoffs erhalten (12% Ausbeute), dessen Morphologie und Struktur sehr voluminös ist. Partikuläre Strukturen werden in diesem Vergleichsbeispiel analog zu Vergleichsbeispiel 2 nicht beobachtet.

Beispiel 4 bis 8

Versuche zur Herstellung von Mikropartikeln aus Stärke, die aus verschiedenen Pflanzen separiert wurde.

500 mg Stärke (Spezifikation siehe Tabelle 1) werden in 2,5 ml Dimethylsulfoxid (DMSO, p.a. von Riedel-de-Haen) bei ca. 70°C gelöst. Es bilden sich keine Lösungen aus. Die Mischungen bilden zähe Gele aus. Diese werden unter Rühren zu 100 ml bidestilliertem Wasser gegeben. Dabei zerfällt das Gel. Die Lösung wird über Nacht bei 5°C aufbewahrt. Es bilden sich stark getrübte Suspensionen mit einer hohen Anzahl großer weißer Flocken aus. Die weitere Aufarbeitung erfolgt wie in Beispiel 1 beschrieben. Die Ergebnisse der Beispiele sind in Tabelle 1 aufgelistet. Bei allen Vergleichsbeispielen 2 bis 8 fällt auf, daß die nicht-linearen Polysaccharide oder sonstigen Ausgangsmaterialien von den Ergebnissen der in Beispiel 1 beschriebenen Erfindung sehr stark abweichen. Es bilden sich ausnahmslos starke Trübungen oder/und große Flocken aus.

Partikulär geformte Strukturen lassen sich nicht beobachten. Darüber hinaus sind die Ausbeuten an Feststoffen in den Vergteichsbeispielen 2 bis 8 deutlich geringer im Vergleich zu Beispiel 1.

Tabelle 1:

| Beispiel | Stärke Typ | Anteil lineares Polysaccharid (%) | Konsistenz der DMSO Lösung | Konsistenz der Suspension nach der Fällung bei 5°C | Auswaage (mg) | Ausbeute (%) |
|---|---|---|---|---|---|---|
| | Ergebnisse der Fällung von verschiedenen Stärke-DMSO Lösungen in Wasser | | | | | |
| 1 | 1,4-α-D-Polyglucan*1 | 100 | klare, niedrigviskose Lösung | feine, milchige Suspension | 311,0 | 62 |
| 2 | Amylose*2 (EGA-Chemie) | 90 - 100 | nach 2d gelöst, hochviskos | feine Suspension mit Flocken | 210,3 | 42 |
| 3 | Amylose *2 (Merck) | 95 - 100 | nach 2d gelöst, in der Wärme hochviskos | feine Suspension mit Flocken | 60,0 | 12 |
| 4 | Kartoffel Toffena™ (Südstärke) | 20 | festes Gel, klar | starke Trübung | nicht abtrennbar (Zentrifuge) | --- |
| 5 | Mais Stärke (Merck) | 20 | zähes Gel | leichte Trübung, große Flocken | 83,8 | 17 |
| 6 | Mais Stärke C (National Starch) | 50 | zähes Gel | starke Trübung, kleine Flocken | 101,7 | 20 |
| 7 | Mais Stärke HVII (National Starch) | 70 | zähes Gel | starke Trübung, kleine Flocken | 211,1 | 42 |
| 8 | Erbsen (Amylose KG) | 70 | zähes Gel, trüb | starke Trübung, große Flocken | 115,9 | 23 |

*1 wasserunlöslich

*2 wasserlöslich

Beispiel 9 a und b

Herstellung von Mikropartikeln aus 1,4-α-D-Polyglucan im großen Maßstab.

a) g 1,4-α-D-Polyglucan werden in 2 Dimethylsulfoxid (DMSO, p.a. von Riedel-de-Haen) bei 60°C innerhalb von 1,5 h gelöst. Dann wird eine Stunde bei Raumtemperatur gerührt. Die Lösung wird in 20 1 bidestilliertem Wasser unter Rühren durch einen Tropftrichter über einen Zeitraum von 2 h hinzugegeben. Der Ansatz wird für 44 h bei 4°C gelagert. Es bildet sich eine feine Suspension aus. Die Partikel werden abgetrennt, indem zunächst der Überstand abdekantiert wird. Der Bodensatz wird aufgeschlämmt und in kleinen Portionen zentrifugiert (Ultrazentrifuge RC5C: je 5 Minuten bei 5000 Umdrehungen pro Minute). Der feste Rückstand wird insgesamt drei Mal mit bidestilliertem Wasser aufgeschlämmt und erneut zentrifugiert. Die Feststoffe werden gesammelt und die Suspension von ca. 1000 ml gefriergetrocknet (Christ Delta 1-24 KD). Es werden 283 g weißer Feststoff isoliert (Ausbeute 71%).

b) Die gesammelten Überstände werden bei einer Temperatur von - 18°C über Nacht verwahrt. Die Aufarbeitung erfolgt wie beschrieben. Es werden weitere 55 g des weißen Feststoffs isoliert (Ausbeute 15%).
Die Gesamtausbeute dieses Prozesses beträgt somit 85% an farblosen Mikropartikeln.

Beispiel 10

Entschwefelung der Mikropartikel.

Zur Abtrennung in den Partikeln verbliebenen Dimethylsulfoxids wird wie folgt vorgegangen. 100 g der 1,4-$\alpha$-D-Polyglucan aus Beispiel 9 werden in 1000 ml entionisiertem Wasser gegeben. Der Ansatz wird für 24 h unter leichtem Schwenken sich selbst überlassen. Die Abtrennung der Partikel erfolgt wie in Beispiel 9 beschrieben (Ultrazentrifuge RC5C: je 15 Minuten, 3000 U/min). Nach der Gefriertrocknung ergibt sich eine Auswaage von 98.3 g (98% Ausbeute). Die Schwefelbestimmung durch Elementaranalyse ergibt folgende Werte (Prüfmethode Verbrennung und IR-Detektion):

| | |
|---|---|
| Schwefelgehalt der Partikel aus Beispiel 9 | 6% +/- 0,1% |
| Schwefelgehalt der Partikel aus Beispiel 10 | < 0,01% |

Beispiel 11

Untersuchungen der Feststoffe aus den Beispielen 1 bis 9 mittels Elektronenmikroskopie.

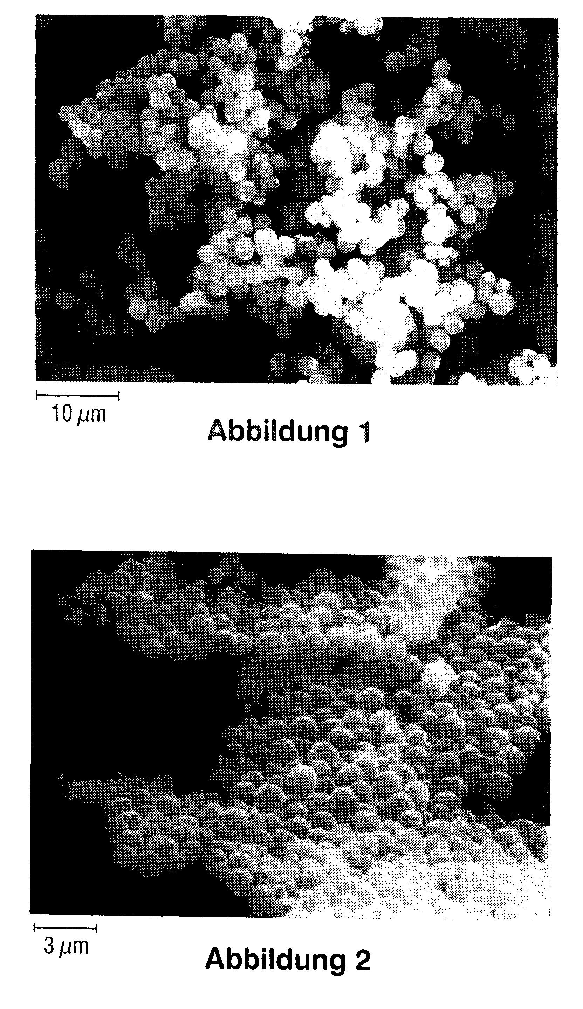

Zur Charakterisierung der Partikel werden Rasterelektronenmikroskopaufnahmen (REM) (Camscan S-4) durchgeführt. Die Ergebnisse der Untersuchung sind in Tabelle 2 festgehalten. Dabei wird deutlich, daß nur bei der Verwendung wasserunlöslicher linearer Polysaccharide (1,4-$\alpha$-D-Polyglucan) sphärisch runde Mikropartikel erhalten werden. Dahingegen führt die Verwendung anderer Ausgangspolymere nur zu voluminösen, watteartigen und nicht-partikulären Morphologien, von denen eine Dispersität nicht bestimmbar ist. Die Struktur der gemäß Beispiel 1 erhaltenen Partikel ist aus Abbildung 1 und 2 ersichtlich.

Tabelle 2:

| Charakterisierung der Feststoffe und Partikel aus den Beispielen 1 bis 3 und 7 bis 9 | | | |
|---|---|---|---|
| Beispiel | Stärke Typ | Anteil lineares Polysaccharid (%) | Aussehen der Partikel |
| 1 | 1,4-$\alpha$-D-Polyglucan[1] | 100 | runde separierte Partikel |
| 2 | Amylose[2](EGA-Chemie) | 90 - 100 | flockig, voluminös, watteartig (d. h. keine separierten Partikel) |
| 3 | Amylose[2] (Merck) | 95 - 100 | flockig, voluminös, watteartig (d. h. keine separierten Partikel) |
| 7 | Mais Hylon VII (National Starch Chemistry) | 70 | flockig, watteartig (d.h. keine separierten Partikel) |
| 8 | Erbsen (Amylose KG) | 70 | flockig, watteartig (d.h. keine separierten Partikel) |
| 9a | 1,4-$\alpha$-D-Polyglucan[1] | 100 | runde separierte Partikel |
| 9b | 1,4-$\alpha$-D-Polyglucan[1] | 100 | runde separierte Partikel |

[1] wasserunlöslich
[2] wasserlöslich

Beispiel 12

Untersuchungen der Größenverteilungen der Partikel aus den Beispielen 1 und 9.

Zur Charakterisierung der Größenverteilungen der Partikel aus den Beispielen 1 und 9 werden Untersuchungen mit einem Mastersizer durchgeführt (Fa. Malvern Instruments). Die Untersuchung erfolgte im Fraunhofer Modus (Auswertung: multimodal, Anzahl) mit einer Dichte von 1.080 $g/cm^3$ und Volumenkonzentration im Bereich von 0,012% bis 0,014%. Die Ergebnisse dieser Untersuchung sind in Tabelle 3 aufgelistet und zeigen die hohe Einheitlichkeit der Mikropartikel.

Beispiel 13

In-vitro-Produktion von 1,4-$\alpha$-D-Polyglucan in einem biokatalytischen Prozeß mit Hilfe von Amylosucrase.

In einem sterilisierten (Dampfsterilisation) 15 l Gefäß werden 10 l einer 20%igen Saccharose-Lösung gegeben. Der Enzymextrakt, Amylosucrase enthaltend, wird in einer Portion zugegeben. Die Enzymaktivität beträgt in diesem Experiment 16 units. Die Apparatur wird mit einem ebenfalls sterilisierten KPG-Rührer versehen. Das Gefäß wird verschlossen und bei 37°C aufbewahrt und gerührt. Bereits nach einer Zeit von wenigen Stunden bildet sich ein weißer Niederschlag. Die Reaktion wird nach einer Zeitdauer von 180 Stunden beendet. Der Niederschlag wird abfiltriert und zur Abtrennung niedermolekularer Zucker fünf Mal mit Wasser gewaschen. Der im Filter verbleibende Rückstand wird bei 40°C im Trockenschrank unter Anlegung eines Vakuums mit Hilfe einer Membranpumpe (Firma Vacuubrand GmbH & Co, CVC 2) getrocknet. Die Masse beträgt 685 g (Ausbeute 69 %). Das so erhaltene 1,4-$\alpha$-D-Polyglucan kann direkt für die Charakterisierung und für die Herstellung von Mikropartikeln eingesetzt werden.

Beispiel 14

Charakterisierung des mit Amylosucrase synthetisierten wasserunlöslichen 1,4-$\alpha$-D-Polyglucans aus Beispiel 13.

Es werden 2 mg des 1,4-$\alpha$-D-Polyglucans aus Beispiel 13 bei Raumtemperatur in Dimethylsulfoxid (DMSO, p.a. von Riedel-de-Haen) gelöst und filtriert (2 µm Filter). Ein Teil der Lösung wird in eine Gelpermeationschromatographie Säule injiziert. Als Elutionsmittel wird DMSO verwendet. Die Signalintensität wird mittels eines RI-Detektors gemessen und gegen Pullulanstandards (Firma Polymer Standard Systems) ausgewertet. Die Flußrate beträgt 1.0 ml pro Minute.
Die Messung ergibt ein Zahlenmittel des Molekulargewichts ($M_n$) von 14.200 g/mol und ein Gewichtsmittel des Molekulargewichts ($M_w$) von 29.500 g/mol. Dies entspricht einer Dispersität von 2,1.

Tabelle 3:

| Charakterisierung der Partikeldurchmesser aus den Beispielen 1 und 9 | | | | | | |
|---|---|---|---|---|---|---|
| Beispiel | Durchmesser | | | Partikelverteilung | | |
| Beispiel No. | $d_n$[*1] (µm) | $d_w$[*2] (µm) | $d_w / d_n$[*3] | d (10%)[*4] (µm) | d (50%)[*5] (µm) | d (90%)[*6] (µm) |
| 1 | 1,282 | 2,692 | 2,100 | 0,991 | 1,263 | 1,776 |
| 9a | 1,664 | 4,184 | 2,541 | 0,873 | 1,504 | 2,624 |
| 9b | 0,945 | 2,345 | 2,481 | 0,587 | 0,871 | 1,399 |

[*1] $d_n$: Zahlenmittelwert des Durchmessers

[*2] $d_w$: Gewichtsmittelwert des Durchmessers

[*3] $d_w / d_n$: Dispersität der Partikeldurchmesser

[*4] d (10%): 10% aller Partikel haben einen kleineren Durchmesser als der angegebene Wert

[*5] d (50%): 50% aller Partikel haben einen kleineren Durchmesser als der angegebene Wert

[*6] d (90%): 90% aller Partikel haben einen kleineren Durchmesser als der angegebene Wert

**Patentansprüche**

1.   Sphärische Mikropartikel mit einem mittleren Durchmesser von 1 nm bis 100 µm, bestehend ganz oder teilweise aus mindestens einem wasserunlöslichen linearen Polysaccharid, wobei die Partikel eine Dispersität in einem Bereich von 1,0 bis 10,0 aufweisen und separiert vorliegen.

**2.** Sphärische Mikropartikel mit einem mittleren Durchmesser von 1 nm bis 100 µm, bestehend ganz oder teilweise aus mindestens einem wasserunlöslichen linearen Polysaccharid, welches in einem biotechnischen Prozeß hergestellt wurde.

**3.** Sphärische Mikropartikel mit einem mittleren Durchmesser von 1 nm bis 100 µm nach Anspruch 2, bestehend ganz oder teilweise aus mindestens einem wasserunlöslichen linearen Polysaccharid, welches durch einen biokatalytischen Prozeß hergestellt wurde.

**4.** Sphärische Mikropartikel mit einem mittleren Durchmesser von 1 nm bis 100 µm nach Anspruch 2, bestehend ganz oder teilweise aus mindestens einem wasserunlöslichen linearen Polysaccharid, welches durch einen fermentativen Prozeß hergestellt wurde.

**5.** Sphärische Mikropartikel nach Anspruch 1, bestehend ganz oder teilweise aus 1,4-$\alpha$-D-Polyglucan.

**6.** Mikropartikel nach Anspruch 5, **dadurch gekennzeichnet, daß** 1,4-$\alpha$-D-Polyglucan mittels eines biokatalytischen Prozesses mit Hilfe von Polysaccharidsynthasen hergestellt wurde.

**7.** Mikropartikel nach Anspruch 5, **dadurch gekennzeichnet, daß** 1,4-$\alpha$-D-Polyglucan mittels eines biokatalytischen Prozesses mit Hilfe von Stärkesynthasen hergestellt wurde.

**8.** Mikropartikel nach Anspruch 5, **dadurch gekennzeichnet, daß** 1,4-$\alpha$-D-Polyglucan mittels eines biokatalytischen Prozesses mit Hilfe von Glykosyltransferasen hergestellt wurde.

**9.** Mikropartikel nach Anspruch 5, **dadurch gekennzeichnet, daß** 1,4-$\alpha$-D-Polyglucan mittels eines biokatalytischen Prozesses mit Hilfe von $\alpha$-1,4-Glucantransferasen hergestellt wurde.

**10.** Mikropartikel nach Anspruch 5, **dadurch gekennzeichnet, daß** 1,4-$\alpha$-D-Polyglucan mittels eines biokatalytischen Prozesses mit Hilfe von Glycogensynthasen hergestellt wurde.

**11.** Mikropartikel nach Anspruch 5, **dadurch gekennzeichnet, daß** 1,4-$\alpha$-D-Polyglucan mittels eines biokatalytischen Prozesses mit Hilfe von Amylosucrasen hergestellt wurde.

**12.** Mikropartikel nach Anspruch 5, **dadurch gekennzeichnet, daß** 1,4-$\alpha$-D-Polygiucan mittels eines biokatalytischen Prozesses mit Hilfe von Phosphorylasen hergestellt wurde.

**13.** Mikropartikel nach Anspruch 1, **dadurch gekennzeichnet, daß** die linearen Polysacchariden durch enzymatische Behandlung von verzweigten oder hochverzweigten Polysacchariden hergestellt wurden.

**14.** Mikropartikel nach mindestens einem der Ansprüche 1 bis 13 mit einem mittleren Durchmesser von 100 nm bis 10 µm, vorzugsweise 1 bis 3 µm.

**15.** Mikropartikel nach einem der Ansprüche 1 bis 14, **gekennzeichnet durch** eine Dispersität der Partikeldurchmesser $d_w$ zu $d_n$ von 1,5 bis 5,0, insbesondere 2,0 bis 2,6.

**16.** Mikropartikel nach mindestens einem der Ansprüche 1 bis 15, **dadurch gekennzeichnet, daß** sie zusätzlich ein oder mehrere, vorzugsweise biologisch abbaubare Polymere enthalten.

**17.** Mikropartikel nach einem oder mehreren der Ansprüche 1 bis 18, **dadurch gekennzeichnet, daß** sie zusätzlich einen oder mehrere Wtrkstoffe enthalten.

**18.** Verfahren zur Herstellung von sphärischen Mikropartikel, welche ganz oder teilweise aus wasserunlöslichen linearen Polysacchariden, insbesondere 1,4-$\alpha$-D-Polyglucan bestehen, durch Lösen des wasserunlöslichen linearen Polysaccharids oder des 1,4-$\alpha$-D-Polyglucans in einem Lösungsmittel, Einbringen der Lösung in ein Fällmittel, Kühlen des dabei entstehenden Gemisches und Abtrennen der gebildeten Mikropartikel.

**19.** Verfahren nach Anspruch 18, **dadurch gekennzeichnet, daß** man Lösung und Fällmittel bei Temperaturen von 20 bis 50°C vermengt und das Gemisch auf Temperaturen von + 10 bis - 10°C, vorzugsweise 5 bis - 5°C kühlt.

**20.** Verfahren nach Anspruch 18 oder 19, **dadurch gekennzeichnet, daß** man als Lösungsmittel Dimethylsulfoxid verwendet.

**21.** Verfahren nach mindestens einem der Ansprüche 18 bis 20, **dadurch gekennzeichnet, daß** man als Fällmittel Wasser oder ein wäßriges Medium verwendet.

**22.** Verfahren nach mindestens einem der Ansprüche 18 bis 21, **dadurch gekennzeichnet, daß** man die Lösung in Gegenwart eines oder mehrerer Polymere, insbesondere biologisch abbaubare Polymere und/oder eines oder mehrerer Wirkstoffe herstellt.

**23.** Verwendung der Mikropartikel nach mindestens einem der Ansprüche 1 bis 17 oder der nach einem Verfahren nach mindestens einem der Ansprüche 18 bis 22 hergestellten Mikropartikel zur kontrollierten Abgabe von Wirkstoffen.

**24.** Verwendung der Mikropartikel nach mindestens einem der Ansprüche 1 bis 17 oder der nach einem Verfahren nach mindestens einem der Ansprüche 18 bis 22 hergestellten Mikropartikel als Standard zur Größenbestimmung von Partikeln.

## Claims

**1.** Spherical microparticles having an average diameter of 1 nm to 100 μm, consisting wholly or partly of at least one water-insoluble linear polysaccharide, the particles having a dispersity in a range from 1.0 to 10.0 and being separate.

**2.** Spherical microparticles having an average diameter of 1 nm to 100 μm, consisting wholly or partly of at least one water-insoluble linear polysaccharide which has been prepared in a biotechnological process.

**3.** Spherical microparticles having an average diameter of 1 nm to 100 μm as claimed in claim 2, consisting wholly or partly of at least one water-insoluble linear polysaccharide which has been prepared by a biocatalytic process.

**4.** Spherical microparticles having an average diameter of 1 nm to 100 μm as claimed in claim 2, consisting wholly or partly of at least one water-insoluble linear polysaccharide which has been prepared by a fermentation process.

**5.** Spherical microparticles as claimed in claim 1, consisting wholly or partly of 1,4-α-D-polyglucan.

**6.** Microparticles as claimed in claim 5, wherein 1,4-α-D-polyglucan has been prepared by a biocatalytic process using polysaccharide synthases.

**7.** Microparticles as claimed in claim 5, wherein 1,4-α-D-polyglucan has been prepared by a biocatalytic process using starch synthases.

**8.** Microparticles as claimed in claim 5, wherein 1,4-α-D-polyglucan has been prepared by a biocatalytic process using glycosyl-transferases.

**9.** Microparticles as claimed in claim 5, wherein 1,4-α-D-polyglucan has been prepared by a biocatalytic process using α-1,4-glucan transferases.

**10.** Microparticles as claimed in claim 5, wherein 1,4-α-D-polyglucan has been prepared by a biocatalytic process using glycogen synthases.

**11.** Microparticles as claimed in claim 5, wherein 1,4-α-D-polyglucan has been prepared by a biocatalytic process using amylosucrases.

**12.** Microparticles as claimed in claim 5, wherein 1,4-α-D-polyglucan has been prepared by a biocatalytic process using phosphorylases.

13. Microparticles as claimed in claim 1, wherein the linear polysaccharides have been prepared by enzymatic treatment of branched or highly branched polysaccharides.

14. Microparticles as claimed in at least one of claims 1 to 13 having an average diameter of 100 nm to 10 μm, preferably 1 to 3 μm.

15. Microparticles as claimed in one of claims 1 to 14, wherein the dispersity of the particle diameters $d_w$ to $d_n$ is 1.5 to 5.0, in particular 2.0 to 2.6.

16. Microparticles as claimed in at least one of claims 1 to 15, which additionally comprise one or more, preferably biodegradable polymers.

17. Microparticles as claimed in one or more of claims 1 to 18, which additionally comprise one or more active substances.

18. A process for preparing spherical microparticles which consist wholly or partly of water-insoluble linear polysaccarides, in particular 1,4-α-D-polyglucan, by dissolving the water-insoluble linear polysaccharide or the 1,4-α-D-polyglucan in a solvent, introducing the solution into a precipitant, cooling the mixture resulting therefrom, and removing the microparticles formed.

19. The process as claimed in claim 18, wherein solution and precipitant are mixed at temperatures from 20 to 50°C, and the mixture is cooled to temperatures from + 10 to - 10°C, preferably 5 to - 5°C.

20. The process as claimed in claim 18 or 19, wherein dimethyl sulfoxide is used as solvent.

21. The process as claimed in at least one of claims 18 to 20, wherein water or an aqueous medium is used as precipitant.

22. The process as claimed in at least one of claims 18 to 21, wherein the solution is prepared in the presence of one or more polymers, in particular biodegradable polymers, and/or of one or more active substances.

23. The use of the microparticles as claimed in at least one of claims 1 to 17 or of the microparticles prepared by a process as claimed in at least one of claims 18 to 22 for the controlled delivery of active substances.

24. The use of the microparticles as claimed in at least one of claims 1 to 17 or of the microparticles prepared by a process as claimed in at least one of claims 18 to 22 as standard for determining the size of particles.

**Revendications**

1. Microparticules sphériques ayant un diamètre moyen de 1 nm à 100 μm, constituées totalement ou partiellement d'au moins un polysaccharide linéaire insoluble dans l'eau, dans lequel les particules présentent une dispersité dans une gamme de 1,0 à 10,0 et se présentent séparées.

2. Microparticules sphériques ayant un diamètre moyen de 1 nm à 100 μm, constituées totalement ou partiellement d'au moins un polysaccharide linéaire insoluble dans l'eau, qui a été préparé dans un procédé de biotechnologie.

3. Microparticules sphériques ayant un diamètre moyen de 1 nm à 100 μm selon la revendication 2, constituées totalement ou partiellement d'au moins un polysaccharide linéaire insoluble dans l'eau, qui a été préparé selon un procédé biocatalytique.

4. Microparticules sphériques ayant un diamètre moyen de 1 nm à 100 μm selon la revendication 2, constituées totalement ou partiellement d'au moins un polysaccharide linéaire insoluble dans l'eau, qui a été préparé au moyen d'un procédé de fermentation.

5. Microparticules sphériques selon la revendication 1, constituées totalement ou partiellement de 1,4-α-D-polyglucane.

**6.** Microparticules selon la revendication 5, **caractérisées en ce que** le 1,4-$\alpha$-D-polyglucane a été préparé au moyen d'un procédé biocatalytique à l'aide de polysaccharide-synthases.

**7.** Microparticules selon la revendication 5, **caractérisées en ce que** le 1,4-$\alpha$-D-polyglucane a été préparé au moyen d'un procédé biocatalytique à l'aide d'amidon-synthases.

**8.** Microparticules selon la revendication 5, **caractérisées en ce que** le 1,4-$\alpha$-D-polyglucane a été préparé au moyen d'un procédé biocatalytique à l'aide de glycosyltransférases.

**9.** Microparticules selon la revendication 5, **caractérisées en ce que** le 1,4-$\alpha$-D-polyglucane a été préparé au moyen d'un procédé biocatalytique à l'aide d'$\alpha$-1,4-glucane-transférases.

**10.** Microparticules selon la revendication 5, **caractérisées en ce que** le 1,4-$\alpha$-D-polyglucane a été préparé au moyen d'un procédé biocatalytique à l'aide de glycogène-synthases.

**11.** Microparticules selon la revendication 5, **caractérisées en ce que** le 1,4-$\alpha$-D-polyglucane a été préparé au moyen d'un procédé biocatalytique à l'aide d'amylosucrases.

**12.** Microparticules selon la revendication 5, **caractérisées en ce que** le 1,4-$\alpha$-D-polyglucane a été préparé au moyen d'un procédé biocatalytique à l'aide de phosphorylases.

**13.** Microparticules selon la revendication 1, **caractérisées en ce que** les polysaccharides linéaires ont été préparés par traitement enzymatique de polysaccharides ramifiés ou hautement ramifiés.

**14.** Microparticules selon au moins une des revendications 1 à 13 ayant un diamètre moyen de 100 nm à 10 $\mu$m, de préférence de 1 à 3 $\mu$m.

**15.** Microparticules selon une des revendications 1 à 14, **caractérisées par** une dispersité des diamètres de particules $d_w/d_n$ de 1,5 à 5,0, en particulier de 2,0 à 2,6.

**16.** Microparticules selon au moins une des revendications 1 à 15, **caractérisées en ce qu'**elles contiennent additionnellement un ou plusieurs polymères, de préférence décomposables biologiquement.

**17.** Microparticules selon une ou plusieurs des revendications 1 à 18, **caractérisées en ce qu'**elles contiennent additionnellement une ou plusieurs substances actives.

**18.** Procédé pour la préparation de microparticules sphériques, qui sont constituées complètement ou partiellement de polysaccharides linéaires insolubles dans l'eau, en particulier de 1,4-$\alpha$-D-polyglucane, par dissolution du polysaccharide linéaire insoluble dans l'eau ou du 1,4-$\alpha$-D-polyglucane dans un solvant, introduction de la solution dans un milieu de précipitation, refroidissement du mélange ainsi obtenu et séparation des microparticules formées.

**19.** Procédé selon la revendication 18, **caractérisé en ce qu'**on mélange une solution et un agent de précipitation à des températures de 20 à 50 °C et **en ce qu'**on refroidit le mélange à des températures de +10 à -10 °C, de préférence de 5 à -5 °C.

**20.** Procédé selon la revendication 18 ou 19, **caractérisé en ce qu'**on utilise comme solvant le diméthylsulfoxyde.

**21.** Procédé selon la revendication 18 à 20, **caractérisé en ce qu'**on utilise comme agent de précipitation l'eau ou un milieu aqueux.

**22.** Procédé selon au moins une des revendications 18 à 21, **caractérisé en ce qu'**on prépare la solution en présence d'un ou de plusieurs polymères, en particulier de polymères décomposables biologiquement et/ou d'une ou plusieurs substances actives.

**23.** Utilisation des microparticules selon au moins une des revendications 1 à 17 ou des microparticules préparées selon un procédé selon au moins une des revendications 18 à 22 pour la libération contrôlée de substances actives.

24. Utilisation des microparticules selon au moins une des revendications 1 à 17 ou des microparticules préparées selon un procédé selon au moins une des revendications 18 à 22 comme étalon pour la détermination des tailles de particules.

Abbildung 1

Abbildung 2